(19) Europäisches Patentamt
European Patent Office
Office européen des brevets

(11) EP 0 415 166 B1

(12) # EUROPÄISCHE PATENTSCHRIFT

(45) Veröffentlichungstag und Bekanntmachung des Hinweises auf die Patenterteilung:
**20.11.1996 Patentblatt 1996/47**

(21) Anmeldenummer: **90115500.2**

(22) Anmeldetag: **13.08.1990**

(51) Int. Cl.[6]: **C07D 513/04**, C07D 513/10 // (C07D513/04, 277:00, 235:00), (C07D513/10, 277:00, 235:00)

(54) **Verfahren zur Herstellung von Cyanhydantoinen**
Process for the preparation of cyanohydantoines
Procédé pour la préparation de cyanohydantoines

(84) Benannte Vertragsstaaten:
**CH DE FR GB IT LI**

(30) Priorität: **26.08.1989 DE 3928263**

(43) Veröffentlichungstag der Anmeldung:
**06.03.1991 Patentblatt 1991/10**

(73) Patentinhaber: **MERCK PATENT GmbH**
**D-64271 Darmstadt (DE)**

(72) Erfinder:
- **Casutt, Michael, Dr.**
  **D-6106 Erzhausen (DE)**
- **Poetsch, Eike, Dr.**
  **D-6109 Mühltal 6 (DE)**

(56) Entgegenhaltungen:
**EP-A- 0 242 686** **US-A- 4 937 351**

- **HELVETICA CHIMICA ACTA, Band 69, Oktober 1986, Seiten 1704-1710, Basel, CH; D. SEEBACH et al.: "Enantiomerenreine Pyrrolidin-Derivate aus trans-4-Hydroxy-L- prolin durch elektrochemische oxidative Decarboxylierung und Titantetrachlorid- vermittelte Umsetzung mit Nukleophilen"**

Bemerkungen:
Die Akte enthält technische Angaben, die nach dem Eingang der Anmeldung eingereicht wurden und die nicht in dieser Patentschrift enthalten sind.

EP 0 415 166 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein verbessertes Verfahren zur Herstellung von chiralen Cyanhydantoinen

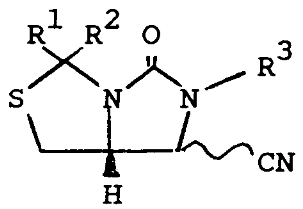

I

wobei

$R^1$ und $R^2$ jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und

$R^3$ H, unsubstituiertes oder durch eine oder mehrere Alkyl-, Alkoxy oder Alk-2-enyl-Gruppen substituiertes Benzyl oder $SiR^4R^5R^6$,

worin

$R^4$, $R^5$ und $R^6$ jeweils unabhängig voneinander geradkettiges oder verzweigtes Alkyl mit 1 bis 8 C-Atomen oder unsubstituiertes oder substituiertes Aryl ist, bedeuten.

Der Erfindung lag die Aufgabe zugrunde, ein verbessertes Verfahren für die Herstellung des bicyclischen Cyanhydantoins der Formel I, einem wertvollen Zwischenprodukt für die Synthese von optisch aktiven D-(+)-Biotin, bereitzustellen, welches die Durchführung einer Racematspaltung und damit das Verwerfen oder Rückführen des unerwünschten Enantiomeren vermeidet.

Verfahren zur stereospezifischen Synthese von D-(+)-Biotin aus Zuckern geeigneter Konfiguration sind bekannt. So wird in Tetrahedron Letters Nr. 32, S. 2765-2766 (1975), als Ausgangsmaterial D-Mannose, in Agric. Biol. Chem. Nr. 42, S. 465 (1978), D-Glucose und in den DE-OS 31 22 562 und DE-OS 33 20 140 D-Arabinose als chirales Ausgangsmaterial verwendet.

Alle diese Verfahren sind jedoch durch eine hohe Zahl von Syntheseschritten mit folglich geringer Gesamtausbeute gekennzeichnet. Die aufgrund ihrer Zuckernatur meist nicht kristallisierbaren Zwischenstufen werden oft nur in unbefriedigender Reinheit erhalten und erfordern, bedingt durch ihre Polyfunktionalität und der damit verbundenen chemischen Labilität die Einhaltung vergleichsweise enger Reaktionsparameter. Eine Reihe von Zuckern ist auch aus natürlichen Quellen nicht zugänglich, was einen hohen Preis zur Folge hat.

Die Verwendung von L-Cystein, wie aus US-4009172, US-4130713, US-4337345 und Journal of the American Chemical Society Nr. 99, S. 7020 (1977) bekannt, vermeidet zwar die Handhabung labiler Zwischenstufen, führt hingegen über insgesamt 18 Reaktionsstufen unter Abtrennung unerwünschter Isomere nur in unbefriedigender Ausbeute zu optisch aktivem D-(+)-Biotin.

Die von Corey et al. (Tetrahedron Letters 29, 57 (1988)) beschriebene Totalsynthese von D-(+)-Biotin aus dem Hydantoin von L-Cystin ergibt über viele Syntheseschritte lediglich 12 % Ausbeute an D-(+)-Biotin und ist somit für die technische Durchführung nicht geeignet.

In einem weiteren Verfahren werden in Journal of the American Chemical Society Nr. 105, S. 5946 (1983) und in der EP-OS 0 094 776 substituierte 3H,5H-Imidazo [1,5-c]-tetrahydrothiazole beschrieben, aus denen nach Racematspaltung optisch aktives Biotin erhalten wird.

Eine sehr elegante Synthese von D-(+)-Biotin aus Cystein bzw. Cystin über das optisch aktive bicyclische Hydantoin-Derivat der Formel I als Zwischenprodukt wurde von E. Poetsch und M. Casutt (Chimia, 41, 148 (1987) und EP-A2-0 242 686/EP-A2-0 243 734) beschrieben.

Der einzige Nachteil dieser Totalsynthese ist, daß die Hydroxygruppe des Alkohols der Formel II in eine aktivierte Estergruppe, welche bei der Umsetzung mit dem Cyanid-Reagenz als leicht abzuspaltende Abgangsgruppe fungiert, überführt werden muß.

Es ist bekannt, daß sich Dialkylacetale mit Trimethylsilylcyanid in die entsprechenden Alkylcyanhydrine überführen lassen, wobei jedoch bei cyclischen Acetalen (z.B. 2-Methoxytetrahydrofuran) regelmäßig die Spaltung des Heterocyclus als unwünschte Nebenreaktion auftritt, z.B. K. Utimoto et al. Tetrahedron 39, No. 6, S. 967-973.

Weiterhin ist bekannt, daß man durch Umsetzung von 1-Acylprolinolethern mit Trimethylsilylcyanid in Gegenwart von Titantetrachlorid 1-Acyl-2-cyanopyrrolidin-Derivate erhält, z.B. P. Renaud, D. Seebach, Helv. Chim. Acta. 69, 1986, 1704-1710.

Da die Herstellung der aktiven Ester aus den Alkoholen der Formel II wie in EP-A2-0242686/0243734 beschrieben teuere, für die großtechnische Produktion schlecht einsetzbare Reagenzien erfordert, deren Umsetzung zu Nebenprodukten führt, die eine aufwendige Aufreinigung der Reaktionsgemische erforderlich macht, da diese Stoffe sonst die Umwelt erheblich belasten würden, bestand weiterhin Bedarf an einem geeigneten Verfahren zur einfachen ökonomischen Herstellung des bicyclischen Cyanhydantoins der Formel I.

Es wurde nun überraschen gefunden, daß das bicyclische Cyanhydantoin der Formel I, das ein wertvolles Zwischenprodukt für die Synthese von optisch aktiven D-(+)-Biotin darstellt durch Umsetzung des Ethers der Formel III mit einem Cyanosilan in Gegenwart einer Lewis-Säure hergestellt werden kann.

Gegenstand der Erfindung ist demnach ein Verfahren zur Herstellung eines Cyanhydantoins der Formel I,

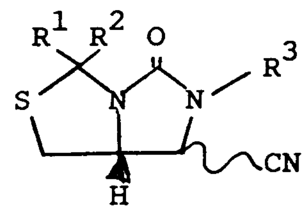

I

wobei

$R^1$ und $R^2$ jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und

$R^3$ H, unsubstituiertes oder durch eine oder mehrere Alkyl-, Alkoxy oder Alk-2-enyl-Gruppen substituiertes Benzyl oder $SiR^4R^5R^6$,

worin

$R^4$, $R^5$ und $R^6$ jeweils unabhängig voneinander geradkettiges oder verzweigtes Alkyl mit 1 bis 8 C-Atomen oder unsubstituiertes oder substituiertes Aryl ist,

bedeuten,
dadurch gekennzeichnet, daß man eine Hydroxy-Verbindung der Formel II,

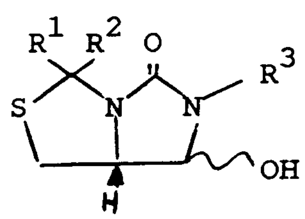

II

wobei $R^1$, $R^2$ und $R^3$ die angegebene Bedeutung besitzen in einen Ether der Formel III,

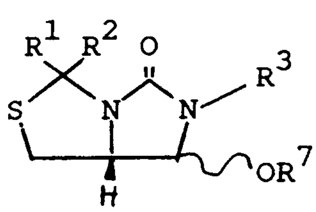

III

wobei $R^1$, $R^2$ und $R^3$ die angegebene Bedeutung besitzen und $R^7$ geradkettiges oder verzweigtes Alkyl mit bis zu 8 C-Atomen ist, und diesen Ether mit einem Cyano-Silan in Gegenwart einer Lewis-Säure umsetzt.

Insbesondere Gegenstand der Erfindung ist ein Verfahren zur Herstellung der Verbindungen der Formel I, dadurch gekennzeichnet, daß man die Hydroxy-Verbindung der Formel II mit einem Alkohol der Formel

$$R^7\text{-OH}$$

in Gegenwart eines wasserentziehenden Mittels umsetzt, sowie ein Verfahren wobei man den Ether der Formel III mit Trimethylsilylcyanid in Gegenwart einer Zinn-, Aluminium- oder Titan-Verbindung umsetzt.

Weiterhin sind die Verbindungen der Formel III

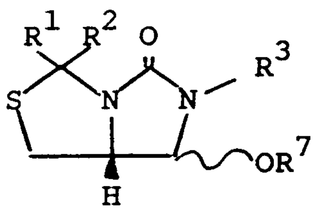

III

worin $R^1$, $R^2$, $R^3$ und $R^7$ die in Anspruch 1 angegebene Bedeutung besitzen, insbesondere worin $R^1$ H, $R^2$ Phenyl, $R^3$ Benzyl und $R^6$ Alkyl mit 1 bis 4 C-Atomen bedeutet, Gegenstand der Erfindung.

Darüberhinaus ist die Verwendung des nach dem erfindungsgemäßen Verfahren hergestellten Cyanhydantoins zur Herstellung von D-(+)-Biotin Gegenstand der Erfindung.

Die Herstellung der Ausgangsverbindungen der Formel II erfolgt nach an sich bekannten Methoden, wie sie in der Literatur z,B. Chimia 31, 148 (1987) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Vor- und nachstehend besitzen $R^1$, $R^2$ und $R^3$ die angegebene Bedeutung, sofern nicht ausdrücklich etwas anderes vermerkt ist.

Die Reste $R^1$ und $R^2$ bedeuten vorzugsweise H, $C_1$-$C_4$-Alkyl, unsubstituiertes oder durch $C_1$-$C_3$-Alkyl und/oder Alkoxy ein oder mehrfach substituiertes Phenyl oder Benzyl, insbesondere bevorzugt sind gleichzeitig $R^1$ = H und $R^2$ = Phenyl.

$R^3$ bedeutet vorzugsweise eine Schutzgruppe für das mit ihr verknüpfte Stickstoffatom, die es ermöglicht, die Verbindung der Formel I in ein D-(+)-Biotin-Derivat, dessen Stickstoffatom mit $R^3$ geschützt ist, mit dem erfindungsgemäßen Verfahren überzuführen, und anschließend diese Schutzgruppe unter milden Bedingungen selektiv abzuspalten.

Solche Schutzgruppen sind dem Fachmann geläufig (z.B. Protective Groups in Organic Chemistry, Plenum Press, New York, 1973) und können nach bekannten Methoden eingeführt und abgespalten werden. Bevorzugte Reste $R^3$ sind beispielsweise Benzyl, Methoxybenzyl, 3,4-Dimethoxybenzyl, 4-Methylbenzyl, Allyl, Methallyl, Crotyl, Trimethylsilyl oder tert.-Butyldimethylsilyl, Diphenylmethyl, Trityl, 9-H-Fluorenyl, 9-Phenyl-9-Fluorenyl, Methoxymethyl.

Die Alkohole der Formel II werden zu den Ethern der Formel III verethert. In den Ethern der Formel III bedeutet $R^7$ vorzugsweise Methyl, Ethyl, n-Propyl, n-Butyl, n-Pentyl, n-Hexyl, Trimethylsilyl, Triisopropylsilyl oder tert. Butyldimethylsilyl sowie weiterhin i-Propyl und tert. Butyl.

Die Herstellung der Alkylether der Formel III ($R^7$ = $C_1$-$C_6$-Alkyl) kann nach den für Veretherungen bekannten und gebräuchlichen Methoden erfolgen, wie sie beispielsweise in Houben-Weyl, Methoden der Organischen Chemie Bd. 6/3, S. 1 ff beschrieben sind. Vorzugsweise werden die entsprechenden Alkohole der Formel $R^7$-OH mit dem Alkohol der Formel III unter Zusatz einer katalytischen Säuremenge oder eines wasserentziehenden Mittels wie z.B. einer Säure wie Schwefelsäure, Phosphorsäure, Chlorwasserstoff oder p-Toluolsulfonsäure, einem Säurederivat wie Trialkylorthoformiaten Phosphorpentoxid, Phosphortrichlorid, oder Phosphoroxychlorid, einem sauren Ionenaustauscher oder Molekularsieben umgesetzt.

Die Veretherung wird in einem geeigneten Lösungsmittel, dem reinen Alkohol $R^7$-OH oder einem Gemisch aus einem geeigneten Lösungsmittel und dem Alkohol $R^7$-OH durchgeführt.

Geeignete Lösungsmittel sind z.B. Benzol, Toluol, Chloroform, Dichlorethan, Diethylether und Tetrahydrofuran. Die Veretherung wird vorzugsweise bei Temperaturen zwischen -20 °C und +150 °C durchgeführt. Insbesondere bevorzugt wird die Veretherung im schwach sauren Milieu, d.h. bei einem pH zwischen 2 und 4, vorzugsweise zwischen 2,5 und 3,5, durchgeführt, um eine Dehydratisierung des Alkohols der Formel III zu unterdrücken.

Die Ether der Formel III werden zur Darstellung der bicyclischen Cyanohydantoinen der Formel I mit einem Cyanosilan, vorzugsweise einem Trialkylsilylcyanid wie z.B. Trimethylsilylcyanid (TMSCN), Triethylsilylcyanid (TESCN), Triisopropylsilylcyanid (TIPSCN) und tert-Butyldimethylsilylcyanid (TBDMSCN) oder einem β-Trialkylsilylpropionitril wie z.B. β-Trimethylsilylpropionitril, in Gegenwart einer Lewis-Säure umgesetzt.

Vorzugsweise werden die Reaktionen in einem inerten Lösungsmittel wie z.B. Dichlormethan oder Toluol durchgeführt.

Geeignete Lewis-Säuren sind Metallsalze wie Aluminium-Zinn-, Zink- und Titanhalogenide, wie z.B. Aluminiumchlorid, Zinntetrachlorid, Zinkbromid und Titantetrachlorid, Borhalogenide wie z.B. Bortrifluorid, Bortrichlorid und Trimethylborat, Zinnester wie z.B. Zinn(IV)-triflat, gemischte Alkylhalogentitanate wie z.B. Triisopropylchlortitanat und Trichlorisopropyltitanat oder Trialkylsilyltriflat und Triisopropylsilyltriflat. Die Reaktionstemperatur liegt üblicherweise zwischen -80° und 150° vorzugsweise zwischen 0° und 80° C. Bei diesen Temperaturen sind die Reaktionen üblicherweise nach 15 Minuten bis 4 Stunden beendet.

Das erfindungsgemäße Verfahren erlaubt somit die Herstellung von optisch aktiven Cyanhydantoins der Formel I, die nach der in EP-A2-0242686/EP-A2-0243734 beschriebenen Methode zu D-(+)-Biotin weiterverarbeitet werden können, auf einfache und stereospezifische Weise in hohen Ausbeuten aus leicht zugänglichen, wohlfeilen Ausgangsstoffen in wenigen Syntheseschritten und stellt damit einen wesentlichen Fortschritt auf dem Gebiet der Biotinsynthese dar.

Die folgenden Beispiele sollen das erfindungsgemäße Verfahren näher erläutern ohne es zu begrenzen.

Die spezifischen Drehwerte sind auf einem Perkin-Elmer Polarimeter im jeweils angegebenen Lösungsmittel gemessen.

Die Säulenchromatographischen Trennungen werden auf Silica 60 230-400 mesh durchgeführt.

## Beispiel 1

(7RS,7aR)-3-Phenyl-6-benzyl-7,7a-dihydro-7-ethoxy-1H, 3H-imidazol[1,5-c]thiazol-5(6H)-on

Eine unter Stickstoff gehaltene Suspension von 195,8 g (0,6 Mol) (7RS,7aR)-3-Phenyl-6-benzyl-7,7a-dihydro-7-hydroxy-1H,3H-imidazol[1,5-c]thiazol-5(6H)-on in 300 ml Ethanol wird mit 93,4 g (0,63 Mol) Triethylorthoformiat sowie 0,5 ml konzentrierter Schwefelsäure versetzt und Minuten bei 60 °C gerührt.

Das Reaktionsgemisch wird anschließend auf Raumtemperatur abgekühlt, mit ethanolischer Kalilauge neutralisiert und unter vermindertem Druck eingeengt. Nach Chromatographie des Rückstands an Kieselgel (Toluol/Ethylacetat 3:1, v/v) erhält man 205, 7 g (7RS,7aR)-3-Phenyl-6-benzyl-7,7a-dihydro-7-ethoxy-1H,3H-imidazol[1,5-c]thiazol-5(6H)-on als farbloses Öl.

$[\alpha]_D^{20}$ = -800°, c = 1 (Methanol)

Analog werden hergestellt

(7RS,7aR)-3-Phenyl-6-benzyl-7,7a-dihydro-7-methoxy-1H,3H-imidazo[1,5-c]thiazol-5(6H)-on
(7RS,7aR)-3-Phenyl-6-benzyl-7,7a-dihydro-7-propoxy-1H,3H-imidazo[1,5-c]thiazol-5(6H)-on
(7RS,7aR)-3-Phenyl-6-benzyl-7,7a-dihydro-7-butoxy-1H,3H-imidazo[1,5-c]thiazol-5(6H)-on
(7RS,7aR)-3-Phenyl-6-benzyl-7,7a-dihydro-7-(i-propoxy)-1H,3H-imidazo[1,5-c]thiazol-5(6H)-on
(7RS,7aR)-3-Phenyl-6-benzyl-7,7a-dihydro-7-hexyloxy-1H,3H-imidazo[1,5-c]thiazol-5(6H)-on
(7RS,7aR)-3,3-Dimethyl-6-benzyl-7,7a-dihydro-7-ethoxy-1H,3H-imidazo[1,5-c]thiazol-5(6H)-on
(7RS,7aR)-3-Methyl-6-benzyl-7,7a-dihydro-7-ethoxy-1H,3H-imidazo[1,5-c]thiazol-5(6H)-on
(7RS,7aR)-3-Ethyl-6-benzyl-7,7a-dihydro-7-ethoxy-1H,3H-imidazo[1,5-c]thiazol-5(6H)-on
(7RS,7aR)-3-Cyclopentyl-6-benzyl-7,7a-dihydro-7-ethoxy-1H,3H-imidazo[1,5-c]thiazol-5(6H)-on
(7RS,7aR)-3-Cyclohexyl-6-benzyl-7,7a-dihydro-7-ethoxy-1H,3H-imidazo[1,5-c]thiazol-5(6H)-on
(7RS,7aR)-3,3-Tetramethylen-6-benzyl-7,7a-dihydro-7-ethoxy-1H,3H-imidazo[1,5-c]thiazol-5(6H)-on
(7RS,7aR)-3,3-Pentamethylen-6-benzyl-7,7a-dihydro-7-ethoxy-1H,3H-imidazo[1,5-c]thiazol-5(6H)-on
(7RS,7aR)-3-(4-Methylphenyl)-6-benzyl-7,7a-dihydro-7-ethoxy-1H,3H-imidazo[1,5-c]thiazol-5(6H)-on
(7RS,7aR)-3-(4-Ethylphenyl)-6-benzyl-7,7a-dihydro-7-ethoxy-1H,3H-imidazo[1,5-c]thiazol-5(6H)-on
(7RS,7aR)-3-(4-Ethoxyphenyl)-6-benzyl-7,7a-dihydro-7-ethoxy-1H,3H-imidazo[1,5-c]thiazol-5(6H)-on
(7RS,7aR)-3-(4-Methoxyphenyl)-6-benzyl-7,7a-dihydro-7-ethoxy-1H,3H-imidazo[1,5-c]thiazol-5(6H)-on
(7RS,7aR)-3-Phenyl-6-(4-methylbenzyl)-7,7a-dihydro-7-ethoxy-1H,3H-imidazo[1,5-c]thiazol-5(6H)-on
(7RS,7aR)-3-Phenyl-6-(4-methoxybenzyl)-7,7a-dihydro-7-ethoxy-1H,3H-imidazo[1,5-c]thiazol-5(6H)-on
(7RS,7aR)-3-Phenyl-6-(4-chlorbenzyl)-7,7a-dihydro-7-ethoxy-1H,3H-imidazo[1,5-c]thiazol-5(6H)-on
(7RS,7aR)-3-Phenyl-6-(4-nitrobenzyl)-7,7a-dihydro-7-ethoxy-1H,3H-imidazo[1,5-c]thiazol-5(6H)-on
(7RS,7aR)-3-Phenyl-6-(1-phenylethyl)-7,7a-dihydro-7-ethoxy-1H,3H-imidazo[1,5-c]thiazol-5(6H)-on
(7RS,7aR)-3-Phenyl-6-allyl-7,7a-dihydro-7-ethoxy-1H,3H-imidazo[1,5-c]thiazol-5(6H)-on
(7RS,7aR)-3-Phenyl-6-trimethylsilyl-7,7a-dihydro-7-ethoxy-1H,3H-imidazo[1,5-c]thiazol-5(6H)-on
(7RS,7aR)-3-Phenyl-6-(tert-butyldimethylsilyl)-7,7a-dihydro-7-ethoxy-1H,3H-imidazo[1,5-c]thiazol-5(6H)-on

Beispiel 2

(7RS,7aR)-3-Phenyl-6-benzyl-7-cyano-7,7a-dihydro-1H,3H-imidazo[1,5-c]thiazol-5(6H)-on

Eine unter Stickstoff gehaltene Lösung von 35,4 g (0,1 Mol) (7RS,7aR)-3-Phenyl-6-benzyl-7,7a-dihydro-7-ethoxy-1H,3H-imidazo[1,5-c]thiazol-5(6H)-on in 150 ml Dichlormethan versetzt man mit 10,9 g (0,11 Mol) Trimethylsilylcyanid, kühlt auf -15 °C ab und tropft 10,4 g (55 mMol) Titan(IV)-chlorid unter kräftigem Rühren zu. Anschließend läßt man ohne Kühlung 3,5 Stunden nachrühren und versetzt bei 0 °C mit 50 ml 1 N Salzsäure. Die organische Phase wird abgetrennt, zweimal mit je 75 ml Wasser gewaschen, über Natriumsulfat getrocknet und eingeengt. Nach Chromatographie des Rückstandes an Kieselgel (Toluol/Ethylacetat 9:1, v/v) erhält man 31,4 g (7RS,7aR)-3-Phenyl-6-benzyl-7-cyano-7,7a-dihydro-1H,3H-imidazo[1,5-c]thiazol-5(6H)-on als Öl.
$[\alpha]_D^{20}$ = -812 °, c = 1 (Methanol)

Anwendungsbeispiel 1

Aus 134,17 g (0,4 mol) (7RS,7aR)-3-Phenyl-6-benzyl-7-cyano-7,7a-dihydro-1H,3H-imidazo[1,5-c]thiazol-5(6H)-on erhält man durch Behandeln mit 1000 ml konzentrierter Salzsäure bei 80 °C für 3 Stunden 127,2 g (7RS,7aR)-3-Phenyl-5(6H)-oxo-6-benzyl-7,7a-dihydro-1H,3H-imidazo[1,5-c]thiazol-7-carbonsäure. Diese wird bei 80 °C in 1500 ml wasserfreier Essigsäure mit 80 g Zinkpulver umgesetzt. Nach Zugabe von 41 g Natriumacetat in Dimethylformamid und Erhitzen auf 110 °C erhält man 88 g (3aS,6aR)-1,3-Dibenzyl-tetrahydro-thieno[3,4-d]imidazol-2(3H),4-dion mit einem Schmelzpunkt von 118 °C, welches z.B. nach DE-PS 20 58 234 oder 23 31 244 in D-(+)-Biotin überführt wird.

Anwendungsbeispiel 2

Das Grignard-Reagenz hergestellt aus 3,68 g (30 mmol) 1-Chlor-4-methoxybutan und 0,97 g (40 mmol) Magnesium wird mit 6,71 g (20 mmol) (7RS,7aR)-3-Phenyl-6-benzyl-7-cyano-7,7a-dihydro-1H,3H-imidazo[1,5-c]thiazol-5(6H)-on, (hergestellt nach Beispiel 2) umgesetzt.

Nach saurer Hydrolyse erhält man 6,6 g (7R,7aR)-3-Phenyl-6-benzyl-7-(5-methoxypentanoyl)-7,7a-dihydro-1H,3H-imidazo[1,5-c]thiazol-5(6H)-on.

Dieses wird analog Anwendungsbeispiel 1 mit 7,06 g (108 mmol) Zinkpulver in 200 ml Essigsäure behandelt und anschließend mit Eisessig/Piperidin auf 100 °C erhitzt.

Nach üblicher Aufarbeitung erhält man 8,1 g (3aS,6aR)-1,3-Dibenzyl-4-(4-methoxybutyliden)-tetrahydrothieno[4,5-d]imidazol-2(3H)-on,, aus welchem nach DE-PS-20 58 243, EP-PS-0 036 030 und EP-PS 0 084 377 D-(+)-Biotin hergestellt wird.

**Patentansprüche**

1. Verfahren zur Herstellung von chiralen Cyanhydantoinen der Formel I,

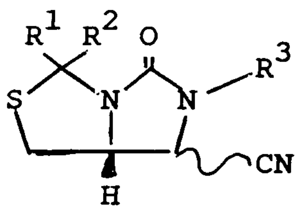

I

wobei

| | |
|---|---|
| $R^1$ und $R^2$ | jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und |
| $R^3$ | H, unsubstituiertes oder durch eine oder mehrere Alkyl-, Alkoxy oder Alk-2-enyl-Gruppen substituiertes Benzyl oder $SiR^4R^5R^6$, |

worin

$R^4$, $R^5$ und $R^6$ jeweils unabhängig voneinander geradkettiges oder verzweigtes Alkyl mit 1 bis 8 C-Atomen oder unsubstituiertes oder substituiertes Aryl ist,

bedeuten,
dadurch gekennzeichnet, daß man eine Hydroxy-Verbindung der Formel II,

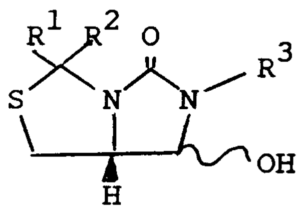

II

wobei $R^1$, $R^2$ und $R^3$ die angegebene Bedeutung besitzen
in einen Ether der Formel III,

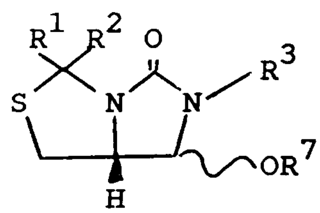

III

wobei $R^1$, $R^2$ und $R^3$ die angegebene Bedeutung besitzen und $R^7$ geradkettiges oder verzweigtes Alkyl mit bis zu 8 C-Atomen ist, und diesen Ether mit einem Cyano-Silan in Gegenwart einer Lewis-Säure umsetzt.

**2.** Verfahren nach Anspruch 1, dadurch gekennzeichnet, daß man die Hydroxy-Verbindung der Formel II mit einem Alkohol der Formel

$$R^7\text{-OH}$$

in Gegenwart eines wasserentziehenden Mittels umsetzt.

**3.** Verfahren nach Anspruch 1 oder 2, dadurch gekennzeichnet, daß man den Ether der Formel III mit Trimethylsilylcyanid in Gegenwart einer Zinn-, Aluminium- oder Titan-Verbindung umsetzt.

**4.** Verbindung der Formel II

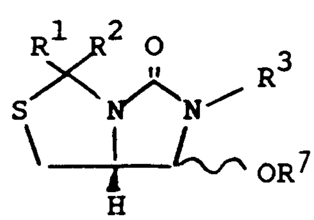

III

worin $R^1$, $R^2$, $R^3$ und $R^7$ die in Anspruch 1 angegebene Bedeutung besitzen.

**5.** Verbindung der Formel III, worin $R^1$ H, $R^2$ Phenyl, $R^3$ Benzyl und $R^7$ Alkyl mit 1 bis 4 C-Atomen bedeutet.

**6.** Verwendung des nach Anspruch 1 hergestellten Cyanhydantoins zur Herstellung von D-(+)-Biotin.

## Claims

1. Process for the preparation of chiral cyanohydantoins of the formula I

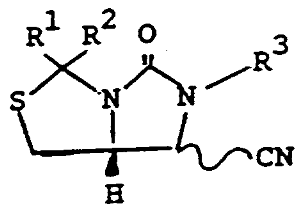

I

in which

$R^1$ and $R^2$, independently of one another, are each H, unsubstituted or substituted alkyl, cycloalkyl, aryl, aralkyl or heteroaryl or together form an unsubstituted alkylene or heteroalkylene, and

$R^3$ is H, benzyl which is unsubstituted or substituted by one or more alkyl, alkoxy or alk-2-enyl groups, or $SiR^4R^5R^6$,

in which

$R^4$, $R^5$ and $R^6$, independently of one another, are each straight-chain or branched alkyl having 1 to 8 carbon atoms or unsubstituted or substituted aryl,

characterized in that a hydroxy compound of the formula II

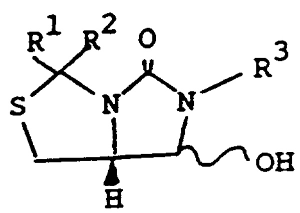

II

in which $R^1$, $R^2$ and $R^3$ are as defined above is converted into an ether of the formula III

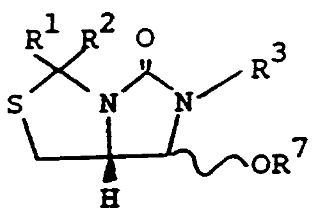

III

in which $R^1$, $R^2$ and $R^3$ are as defined above and $R^7$ is a straight-chain or branched alkyl having up to 8 carbon atoms, and this ether is reacted with a cyano-silane in the presence of a Lewis acid.

2. Process according to Claim 1, characterized in that the hydroxy compound of the formula II is reacted with an alcohol of the formula

$R^7\text{-}OH$

in the presence of a dehydrating agent.

3. Process according to Claim 1 or 2, characterized in that the ether of the formula III is reacted with trimethylsilyl cyanide in the presence of a compound of tin, aluminium or titanium.

4. Compound of the formula III

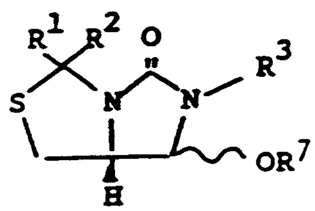

III

in which $R^1$, $R^2$, $R^3$ and $R^7$ are as defined in Claim 1.

5. Compound of the formula III, in which $R^1$ is H, $R^2$ is phenyl, $R^3$ is benzyl and $R^7$ is alkyl having 1 to 4 carbon atoms.

6. Use of the cyanohydantoin prepared according to Claim 1 for the preparation of D-(+)-biotin.

**Revendications**

1. Procédé de préparation de cyanhydantoïnes chirales de formule I

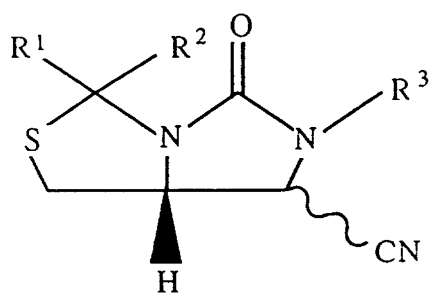

(I)

dans laquelle

$R^1$ et $R^2$ représentent chacun, indépendamment l'un de l'autre, H, un groupe alkyle, cycloalkyle aryle, aralkyle ou hétéroaryle non substitué ou substitué ou forment ensemble un groupe alkylène ou hétéroalkylène non substitué et

$R^3$ représente H, un groupe benzyle non substitué ou substitué par un ou plusieurs groupes alkyle, alcoxy ou alca-2-ényle, ou un groupe $SiR^4R^5R^6$

dans lequel

$R^4$, $R^5$ et $R^6$ représentent chacun, indépendamment les uns des autres, un groupe alkyle à chaîne droite ou ramifiée en $C_1$-$C_8$ ou un groupe aryle non substitué ou substitué,

caractérisé en ce que l'on convertit un dérivé hydroxylé de formule II:

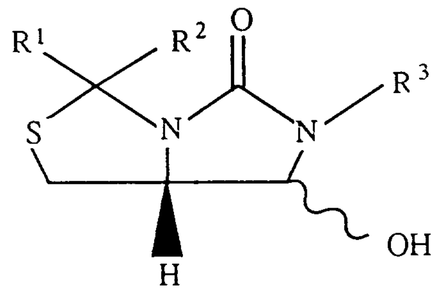

(II)

dans laquelle

$R^1$, $R^2$ et $R^3$ ont les significations indiquées ci-dessus,

en un éther de formule III

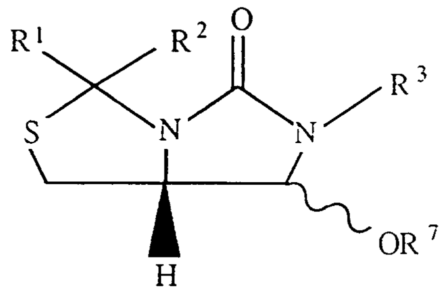

(III)

dans laquelle
$R^1$, $R^2$ et $R^3$ ont les significations indiquées ci-dessus et $R^7$ représente un groupe alkyle à chaîne droite ou ramifiée contenant jusqu'à huit atomes de carbone, et on fait réagir cet éther avec un cyanosilane en présence d'un acide de Lewis.

**2.** Procédé selon la revendication 1, caractérisé en ce que l'on fait réagir le dérivé hydroxylé de formule II avec un alcool de formule

$$R^7\text{-OH}$$

en présence d'un agent déshydratant.

**3.** Procédé selon la revendication 1 ou 2, caractérisé en ce que l'on fait réagir l'éther de formule III avec le cyanure de triméthylsilyle en présence d'un dérivé de l'étain, de l'aluminium ou du titane.

**4.** Composé de formule III

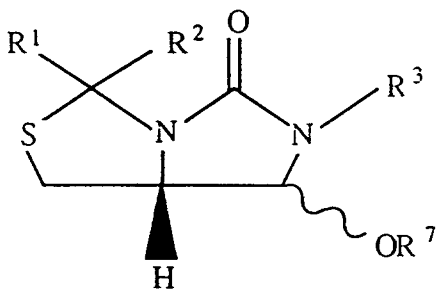

(III)

dans laquelle
$R^1$, $R^2$, $R^3$ et $R^7$ ont les significations indiquées dans la revendication 1.

**5.** Composé de formule III dans laquelle $R^1$ représente H, $R^2$ un groupe phényle, $R^3$ un groupe benzyle et $R^7$ un groupe alkyle en $C_1$-$C_4$.

**6.** Utilisation de la cyanohydantoïne préparée selon la revendication 1 pour la préparation de la D-(+)-biotine.